# FASCICULE DE BREVET EUROPEEN

(11) **EP 0 798 012 B1**
(45) Date de publication et mention de la délivrance du brevet: **17.10.2001**
(21) Numéro de dépôt: 97400643.9
(22) Date de dépôt: 21.03.1997
(51) Int. Cl.: A61M 27/00

(54) **Valve pour le traitement de l'hydrocéphalie**
Ventil zur Behandlung von Hydrocephalus
Valve for the treatment of hydrocephalus

(30) Priorité: 26.03.1996 FR 9603726
(43) Date de publication de la demande: 01.10.1997
(73) Titulaire: CORDIS S.A., F-91170 Viry-Châtillon (FR)
(72) Inventeur: Lecuyer, Alain, 06130 Grasse (FR); Sainte-Rose, Christian, Prof., 92170 Vanves (FR)
(74) Mandataire: Geismar, Thierry

(56) Documents cités:
- FR-A- 2 698 535
- US-A- 4 781 672
- US-A- 5 520 632

## Description

La présente invention concerne une valve pour le traitement de l'hydrocéphalie, et plus particulièrement une telle valve du type comprenant:
- un boîtier formant une cavité;
- une membrane de séparation montée à sa périphérie sur le boîtier, pourvue d'un orifice et délimitant dans ladite cavité une chambre amont et une chambre aval, ladite membrane ayant une première et une deuxième face;
- ledit boîtier formant, dans la chambre amont, un siège pour la membrane, ledit siège entourant ledit orifice;
- une tige de section variable agencée pour pénétrer dans ledit orifice;
- ledit boîtier étant muni d'une ouverture recevant un bouchon dont ladite tige est montée solidaire.

On connaît déjà de telles valves, notamment par le document US-A-4781672.

Les valves de ce type, possèdent de façon connue une caractéristique de fonctionnement en plusieurs zones. Dans un premier temps, aucun écoulement ne se produit tant que la pression différentielle entre la chambre amont et la chambre aval est insuffisante pour décoller la membrane de son siège.

Puis, le profil de la tige est tel que l'on commence à avoir une zone dans laquelle la pression différentielle reste presque constante pour un débit qui croît rapidement. Arrivé à un certain débit, qui est le débit de régulation de la valve, la pression augmente à débit sensiblement constant.

Enfin, au delà d'un certain seuil de pression différentielle, l'extrémité libre de la tige sort de l'orifice de la membrane. Il en résulte une pression différentielle maximum, pratiquement indépendante du débit qui croît à la demande.

Dans les valves connues de ce type, le boîtier est réalisé en deux demi-boîtiers qui pincent la périphérie de la membrane lors de leur assemblage, préalablement à leur collage. Le demi-boîtier supérieur est lui-même ouvert à sa partie supérieure, son ouverture étant filetée intérieurement.

Le bouchon est fileté extérieurement et reçoit la tige en relation fixe. Il est ensuite mis en place dans l'ouverture du demi-boîtier supérieur, et vissé plus ou moins profondément de manière que la tige s'enfonce de façon convenable dans l'orifice de la membrane et que l'on obtienne la caractéristique pression/débit désirée.

Bien que donnant satisfaction, ces valves présentent des inconvénients résultant du fait qu'elles sont d'une fabrication, et surtout d'un réglage, complexes qu'il est souhaitable de simplifier.

La présente invention vise à pallier ces inconvénients.

A cet effet, l'invention a pour objet une valve pour le traitement de l'hydrocéphalie, du type rappelé ci-dessus, caractérisée par le fait que:
- le boîtier est réalisé en trois parties, un premier et un deuxième corps qui pincent la périphérie de la membrane lors de leur assemblage, et un capuchon de fond qui, avec le deuxième corps et la deuxième face de la membrane, délimite l'une des chambres;
- et que l'ouverture est formée dans le premier corps, le bouchon y étant adapté de façon fixe pour délimiter l'autre chambre avec ledit premier corps et la première face de la membrane.

Les différentes parties du boîtier, le capuchon de fond et le bouchon peuvent ainsi être simplement emmanchés, et éventuellement collés, le réglage pouvant s'effectuer au cours du montage, et non plus après le montage par vissage du bouchon comme dans l'art antérieur.

Plus particulièrement, le bouchon et la tige qui en est solidaire peuvent être mis en place avant le capuchon de fond, et le réglage peut être effectué de toute manière convenable à travers l'emplacement laissé libre du fond. Le fond est alors monté après le réglage.

Dans un mode de réalisation particulier, la chambre délimitée par le bouchon, le premier corps et la première face de la membrane est la chambre amont, la chambre aval étant délimitée par le capuchon de fond, le deuxième corps et la deuxième face de la membrane.

Avantageusement, le corps délimitant la chambre aval possède une surface d'appui conique pour la membrane dans sa position où la valve est complètement ouverte.

Ainsi, en cas de forte surpression, la membrane vient en appui sur cette surface conique, ce qui évite tout risque d'endommagement.

Dans un mode de réalisation préféré, le corps délimitant la chambre amont possède à son extérieur un canal sensiblement circulaire par lequel se fait l'admission du fluide à drainer, ledit canal communiquant par une pluralité de trous avec ladite chambre amont.

Ce canal circulaire permet une homogénéisation du flux d'entrée dans la valve, le fluide pénétrant dans la chambre amont par l'ensemble de trous qui sont répartis à la périphérie de la membrane.

On décrira maintenant, à titre d'exemple non limitatif, un mode de réalisation particulier de l'invention, en référence aux dessins schématiques annexés dans lesquels:
- la figure 1 est une vue en coupe d'un ensemble de drainage pour le traitement de l'hydrocéphalie, comportant une valve selon l'invention;
- la figure 2 est une vue en coupe dans les mêmes plans, à plus grande échelle, de la valve représentée à la figure 1;
- les figures 3a et 3b constituent, lorsque la figure 3a est placée au-dessus de la figure 3b, une vue en perspective éclatée de la valve représentée à la figure 2;

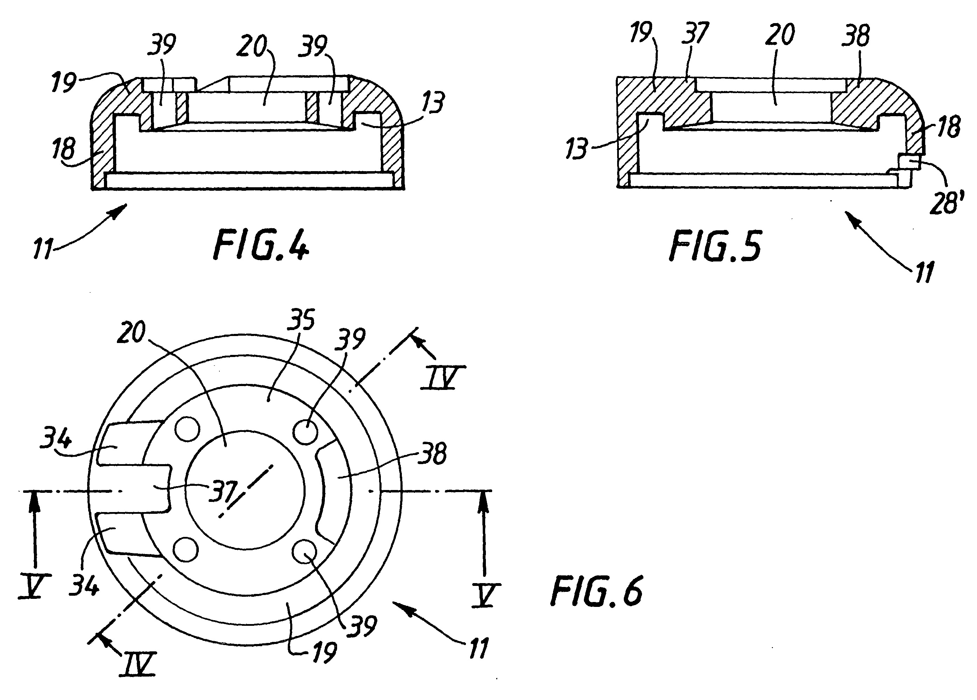

- la figure 4 est une vue en coupe selon la ligne IV-IV de la figure 6, du corps de boîtier supérieur de la valve de la figure 2;
- la figure 5 est une vue en coupe selon la ligne V-V de la figure 6;
- la figure 6 est une vue de dessus du corps de boîtier des figures 4 et 5;

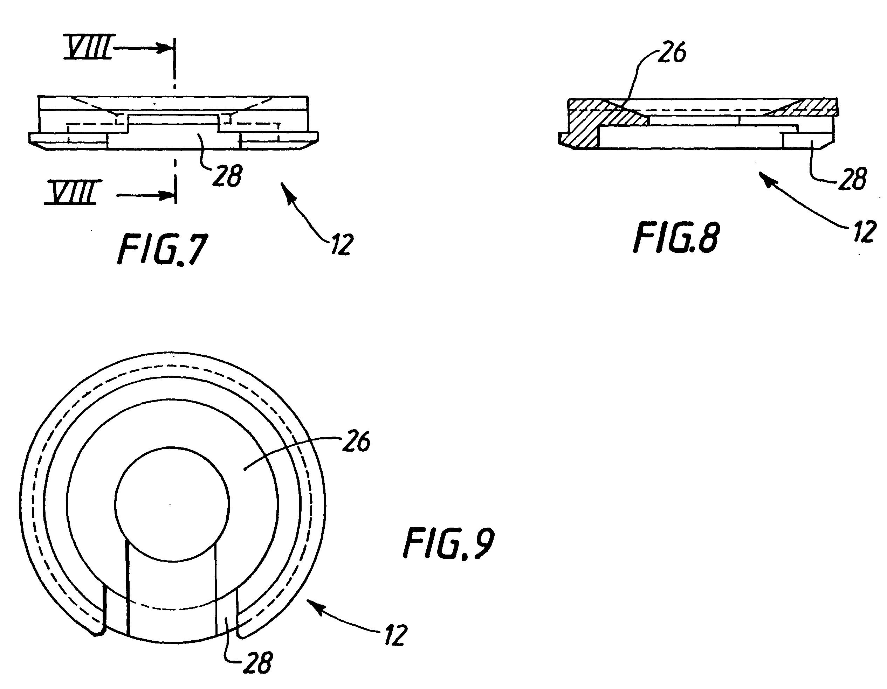

- la figure 7 est une vue de face du corps de boîtier inférieur de la valve de la figure 2;
- la figure 8 est une vue en coupe selon la ligne VIII-VIII de la figure 7;
- la figure 9 est une vue de dessus du corps de boîtier des figures 7 et 8;

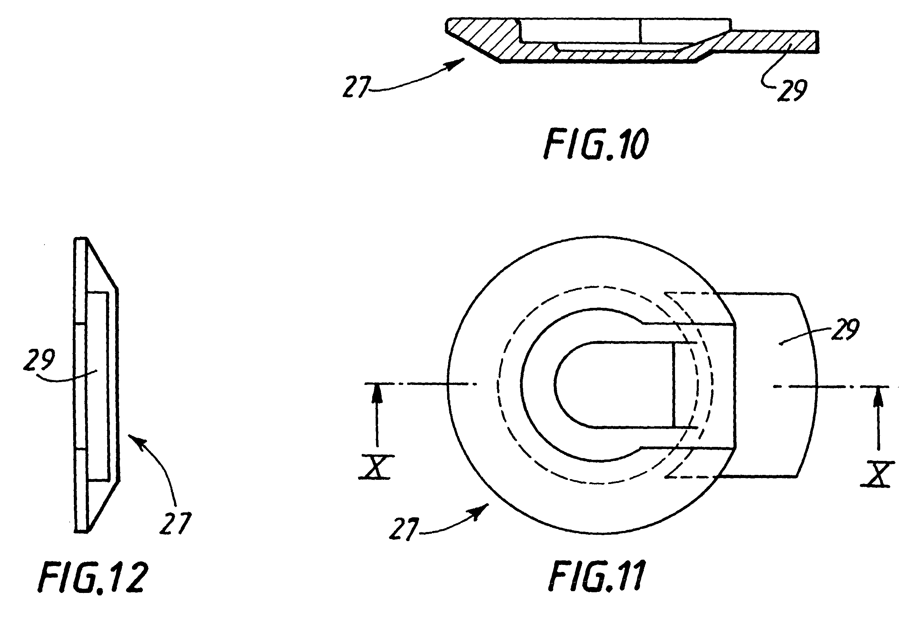

- la figure 10 est une vue en coupe selon la ligne X-X de la figure 11, du capuchon de fond de la valve de la figure 2;
- la figure 11 est une vue de dessus du capuchon de fond de la figure 10; et
- la figure 12 est une vue de droite du capuchon de fond des figures 10 et 11.

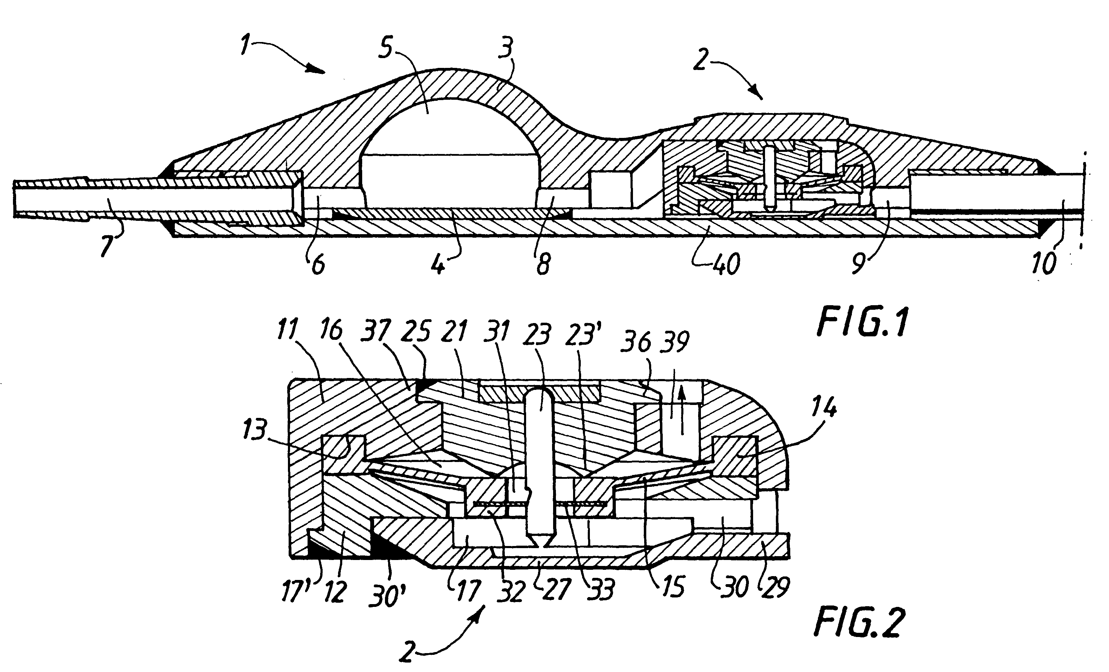

On voit à la figure 1 un ensemble constitué par un site d'injection 1 et une valve 2 selon l'invention.

Le site d'injection comprend pour l'essentiel un dôme 3 en matière synthétique souple et perçable par une aiguille hypodermique, délimitant avec un fond 4 une chambre 5 raccordée par un canal amont 6 à un raccord 7 de cathéter, et par un canal aval 8 à l'entrée dans la valve 2. Un cathéter non représenté a une de ses extrémités montée sur le raccord 7 et son autre extrémité est libre pour être guidée jusqu'au ventricule à drainer. La sortie de la valve 2 est raccordée par un canal 9 à un cathéter de drainage 10 dont l'extrémité libre peut être guidée jusque dans la zone de drainage.

Le site d'injection 5 permet, en écrasant le canal aval 8, d'effectuer une injection dans le ventricule et, en écrasant le canal amont 6, de nettoyer la valve 2.

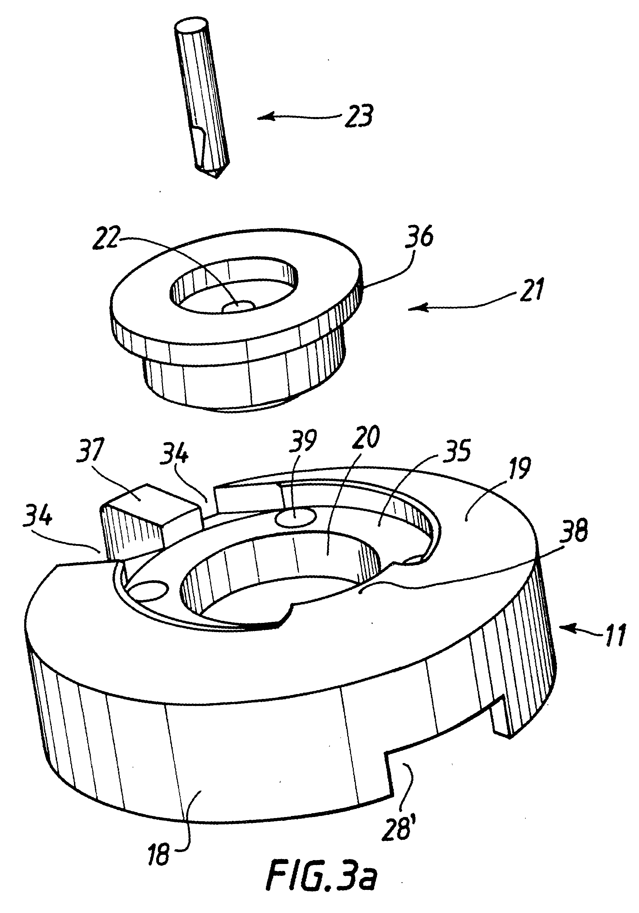

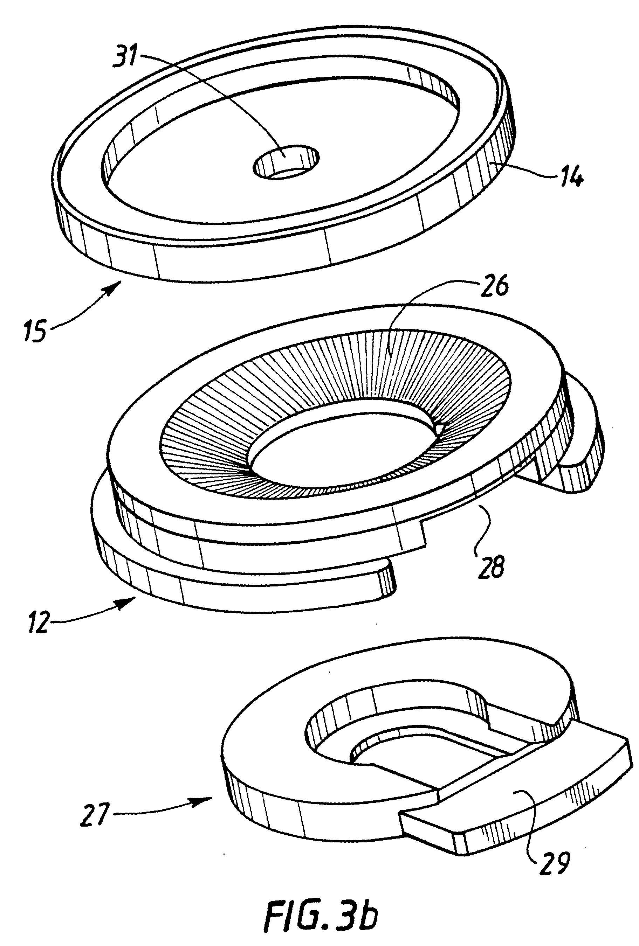

Si l'on se réfère maintenant aux figures 2 et 3, on voit que la valve 2 comprend un corps supérieur 11 et un corps inférieur 12. Le corps supérieur 11 forme une rainure annulaire 13 pour recevoir un bourrelet de bordure 14 d'une membrane souple 15 en forme de disque. Le corps supérieur 11 et la membrane 15 délimitent une chambre amont 16 et le corps inférieur 12 et la membrane 15 délimitent une chambre aval 17. Les chambres amont et aval sont donc séparées par la membrane 15. Des points de colle 17' maintiennent les deux corps ensembles.

Le corps supérieur 11 possède une forme générale de coupelle avec une paroi latérale 18 sensiblement cylindrique et un fond 19 dans lequel est formé un orifice circulaire 20. L'orifice 20 est fermé par un bouchon 21 portant, dans un alésage central 22, une tige usinée 23. En outre le bouchon 21 forme, autour de la tige 23, un siège 23' pour la membrane 15 qui s'y applique tant que la pression différentielle entre les chambres 16 et 17 reste inférieure à un seuil prédéterminé. Une résine 24 maintient la tige 23 dans l'alésage 22 et des points de colle 25 maintiennent le bouchon 21 dans l'orifice 20.

Le corps inférieur 12 est de forme générale annulaire avec une surface supérieure d'appui 26, en vis-à-vis de la membrane 15, sensiblement en tronc de cône et striée. Un capuchon de fond 27 s'emboîte dans le corps inférieur 12 pour fermer la chambre aval 17. Une découpe 28 du corps inférieur 12 délimite, avec une découpe 28' du corps supérieur 11 et un prolongement 29 du capuchon 27, un canal 30 de sortie de la valve 2. Des points de colle 30' maintiennent le capuchon de fond 27 sur le corps inférieur 12.

La membrane 15 possède un orifice central 31 bordé par un bourrelet 32. Lorsque le bouchon 21 est en place, l'extrémité usinée de la tige 23 traverse l'orifice. Une rondelle annulaire 33 est engagée dans une rainure circulaire formée dans la paroi cylindrique intérieure du bourrelet 32.

L'entrée du fluide dans la valve s'effectue par deux canaux 34 latéraux formés dans la paroi du corps supérieur 11. Ces canaux 34 débouchent dans un canal annulaire 35 obstrué, en deux points diamétralement opposés, par un rebord 36 du bouchon 21 et par deux saillies 37 et 38 du corps supérieur 11. Le canal 35 forme donc deux embranchements. Chacun de ces embranchements communique avec la chambre amont par deux trous 39 formés dans le fond 19 du corps supérieur 11.

Le montage des divers éléments qui viennent d'être décrits s'effectue dans un ordre approprié, mais le capuchon de fond 27 est monté en dernier. Ainsi, alors que ce capuchon n'est pas encore en place, il est possible de réaliser le réglage de la valve, et plus particulièrement le centrage de la rondelle 33 par rapport à l'axe de la tige 23.

Une fois la valve 2 complètement montée, elle est mise en place dans un logement prévu à cet effet d'une pièce en matière plastique formant également le dôme 3 du site d'injection. Puis, une semelle 40, le raccord 7 et le cathéter sont à leur tour mis en place pour terminer l'ensemble.

La valve qui vient d'être décrite fonctionne comme les valves de type connu et comme cela a été rappelé ci-dessus.

## Revendications

1. Valve pour le traitement de l'hydrocéphalie comprenant:
- un boîtier formant une cavité;
- une membrane de séparation (15) montée à sa périphérie sur le boîtier, pourvue d'un orifice (31) et délimitant dans ladite cavité une chambre amont (16) et une chambre aval (17);
- ledit boîtier formant dans la chambre amont un siège (23') pour la membrane, ledit siège entourant ledit orifice; et
- une tige (23) de section variable agencée pour pénétrer dans ledit orifice;
- ledit boîtier étant muni d'une ouverture (20) recevant un bouchon (21) dont ladite tige est montée solidaire;
- le boîtier étant réalisé en trois parties, un premier (11) et un deuxième (12) corps qui pincent la périphérie de la membrane lors de leur assemblage;
- ladite ouverture étant formée dans le premier corps, ledit bouchon y étant adapté de façon fixe pour délimiter l'autre chambre avec ledit premier corps et la première face de la membrane;
**caractérisée par le fait que**:
- la troisième partie du boîtier est un capuchon de fond (27) qui, avec le deuxième corps et la deuxième face de la membrane, délimite l'une des chambres.

2. Valve selon la revendication 1, dans laquelle la chambre délimitée par le bouchon, le premier corps et la première face de la membrane est la chambre amont (16), la chambre aval (17) étant délimitée par le capuchon de fond, le deuxième corps et la deuxième face de la membrane.

3. Valve selon l'une quelconque des revendications 1 et 2, dans laquelle le corps délimitant la chambre aval possède une surface d'appui conique (26) pour la membrane dans sa position où la valve est complètement ouverte.

4. Valve selon l'une quelconque des revendications 1 à 3, dans laquelle le corps (11) délimitant la chambre amont (16) possède à son extérieur un canal (35) sensiblement circulaire par lequel se fait l'admission du fluide à drainer, ledit canal communiquant par une pluralité de trous (39) avec ladite chambre amont.

## Patentansprüche

1. Ventil für die Behandlung von Hydrozephalie mit :
- einem einen Hohlraum bildenden Gehäuse,
- einer Trennmembran (15), die an ihrem Umfang am Gehäuse montiert ist, eine Öffnung (31) aufweist und in dem besagten Hohlraum eine vorgelagerte Kammer (16) und eine nachgelagerte Kammer (17) abgrenzt;
- wobei das besagte Gehäuse in der vorgelagerten Kammer einen Sitz (23') für die Membran bildet, wobei der besagte Sitz die besagte Öffnung umgibt; und
- einer Stange (23) mit variablem Querschnitt, die in die besagte Öffnung eintreten soll;
- wobei das besagte Gehäuse eine Öffnung (20) für einen Verschluss (21) aufweist, mit dem die besagte Stange fest verbunden ist;
- wobei das Gehäuse aus drei Teilen besteht, einem ersten (11) und einem zweiten (12) Körper, die den Umfang der Membran bei ihrem Zusammenbau umklammern;
- wobei die besagte Öffnung im ersten Körper ausgebildet ist, wobei der besagte Verschluss dort fest angepasst ist, um die andere Kammer mit dem besagten ersten Körper und der ersten Seite der Membran abzugrenzen;
**dadurch gekennzeichnet, dass** der dritte Teil des Gehäuses eine Bodenkappe (27) ist, die mit dem zweiten Körper und der zweiten Seite der Membran eine der Kammern abgrenzt.

2. Ventil nach Anspruch 1, bei der die vom Verschluss, dem ersten Körper und der ersten Seite der Membran abgegrenzte Kammer die vorgelagerte Kammer (16) ist, wobei die nachgelagerte Kammer (17) von der Bodenkappe, dem zweiten Körper und der zweiten Seite der Membran abgegrenzt wird.

3. Ventil nach einem der Ansprüche 1 und 2, bei der der die nachgelagerte Kammer abgrenzende Körper eine konische Stützfläche (26) für die Membran in ihrer Position besitzt, in der das Ventil völlig geöffnet ist.

4. Ventil nach einem der Ansprüche 1 bis 3, bei der der die vorgelagerte Kammer (16) abgrenzende Körper (11) an seiner Außenseite einen etwa kreisförmigen Kanal (35) besitzt, durch den die zu drainierende Flüssigkeit eintritt, wobei der besagte Kanal über eine Vielzahl von Löchern (39) mit der besagten vorgelagerten Kammer verbunden ist.

## Claims

1. A valve for the treatment of hydrocephalus comprising:
- a casing forming a cavity;
- a separation membrane (15) mounted at its periphery on the casing, provided with an orifice (31) and delimiting in the said cavity an upstream chamber (16) and a downstream chamber (17);
- the said casing forming in the upstream chamber a seat (23') for the membrane, the said seat surrounding the said orifice; and
- a rod (23) with a variable cross-section arranged to enter the said orifice;
- the said casing being provided with an opening (20) receiving a plug (21) on which the said rod is integrally mounted;
- the casing being produced in three parts, a first (11) and a second (12) body which grip the periphery of the membrane when they are assembled;
- the said opening being formed in the first body, the said plug being adapted thereto fixedly in order to delimit the other chamber with the said first body and the first face of the membrane,
**characterised by** the fact that:
- the third part of the casing is a base cap (27) which, with the second body and the second face of the membrane, delimits one of the chambers.

2. A valve according to Claim 1, in which the chamber delimited by the plug, the first body and the first face of the membrane is the upstream chamber (16), the downstream chamber (17) being delimited by the base cap, the second body and the second face of the membrane.

3. A valve according to either one of Claims 1 and 2, in which the body delimiting the downstream chamber has a conical support surface (26) for the membrane in its position in which the valve is completely open.

4. A valve according to any one of Claims 1 to 3, in which the body (11) delimiting the upstream chamber (16) has on its outside a substantially circular channel (35) through which admission of the fluid to be drained takes place, the said channel communicating with the said upstream chamber through a plurality of holes (39).
